# EUROPEAN PATENT APPLICATION

(11) **EP 4 834 205 A1**
(43) Date of publication of application: **04.11.2026**
(21) Application number: 25173718.5
(22) Date of filing: 30.04.2025
(51) Int. Cl.: A61B 5/00, A61B 5/024, A61B 5/1455

(54) **ELECTRONIC DEVICE AND METHOD FOR SENSING A SPECTRAL RESPONSE FROM A SKIN**

(71) Applicant: Imec VZW, 3001 Leuven (BE)
(72) Inventor: Pejovic, Vladimir, 3000 Leuven (BE); Malinowski, Pawel, 3001 Leuven (BE)
(74) Representative: Patent Department IMEC

(57) **Abstract**

An electronic device for sensing a spectral response from a skin; the device comprising an emitter configured for emitting broadband light towards the skin; and an array of thin-film microcavity detectors configured for detecting at least two different respective narrowband portions of the broadband light after transmission through or reflection from the skin; wherein each narrowband portion spans a smaller wavelength range than the broadband light.

## Description

### Technical field

The present disclosure generally relates to electronic devices for spectral sensing. Particular embodiments relate to an electronic device for sensing a spectral response from a skin and to a method for sensing a spectral response from a skin.

### Background

It is known that light can considerably penetrate (i.e. be transmitted through) skin tissue, especially at longer wavelengths, and that this can be used for various applications. For example, vein patterns in human flesh can be detected for more advanced biometric identification compared to today's fingerprint sensors, which can give erroneous readings and can be fooled.

Probably the most widely used application of this technology is optical on-skin (therefore non-invasive) biomarker sensing, i.e. the use of light to detect and/or monitor physiological parameters of body tissue. This technology is also known as photoplethysmography (PPG) and is today ubiquitous, finding application in many medical and consumer devices.

PPG typically utilizes a combination of light emitters (usually light-emitting diodes, LEDs) and light detectors (also called photodetectors, PDs) to measure one or more physiological parameters, such as for example heart rate, blood oxygenation, etc. Light is emitted and is subsequently transmitted through (i.e. passes through) a portion of a body tissue. The light is attenuated within said (portion of) body tissue by different molecules found in skin and bodily fluids. After transmission through the (portion of) body tissue, the light can be detected by a photodetector upon exiting the tissue.

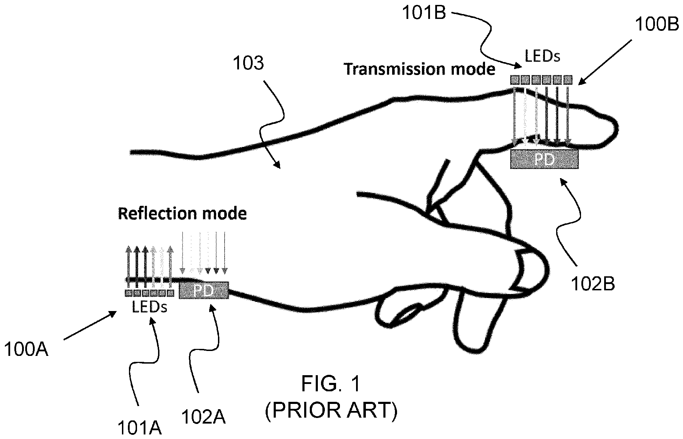

Light emitters and detectors can be arranged on the same side of the measurement site, which is for example convenient for sensing on a wrist. This arrangement can be called a reflection mode 100A of operation since the photodetector collects photons reflected and backscattered from within the tissue. This is illustrated in Figure 1 as reflection mode 100A: both the light emitters (LEDs) and the photodetectors (PD) are situated at the same side of the measurement site (in this example a human hand).

In principle, using one or more reflective elements, the light emitters and photodetectors can be arranged at an angle to each other in relation to the measurement site. For example, the light emitters can be arranged at the side of the measurement site while the photodetectors can be arranged at the bottom of the measurement site, such that the light emitters and the photodetectors are at an angle to each other between 0 and 180 degrees, e.g. 90 degrees.

Alternatively, the light emitters and the photodetectors can be positioned on opposite sides of the measurement site, which may be termed a transmission mode 100B of operation. This transmission mode 100B is often used for sensing on fingers, because fingers represent a relatively small body element through which a significant part of the light emitted from the light emitters can reach the photodetectors. This is illustrated in Figure 1 as transmission mode 100B, wherein the light emitters (LEDs) are situations on one side (in this example the top side) of the measurement site (in this example the finger), and the photodetectors (PD) are situated on the opposite side (in this example the bottom side) of the measurement site (i.e. the finger).

### Summary of certain aspects

Various known PPG solutions are not integrated, in the sense of the combination of multiple components or functions onto a single substrate or chip. Such electronic integration in general allows for miniaturization, improved performance, and increased efficiency compared to discrete components. It is a specific problem for various known PPG solutions that each additional discrete LED increases the system size, cost and power consumption.

Moreover, various known PPG solutions either don't cover the short-wavelength infrared, SWIR, range at all, or not effectively, or not efficiently (in terms of cost or power or both). The commercially available selection of SWIR LEDs is rather limited, and those SWIR LEDs typically have rather broad emission spectra with a significant overlap. Therefore, a high spectral resolution is typically not possible. Moreover, such conventional SWIR components can be very expensive.

It is currently the belief of the inventors that integration of fundamentally different solutions, e.g. lasers on silicon, may prove to be very complicated.

It is an aim of various embodiments according to the present disclosure to alleviate one or more of the above-mentioned problems. Particular embodiments according to the present disclosure may aim to provide an integrated, effective, and/or efficient PPG solution. Further advantages of specific embodiments will be discussed hereinbelow.

According to a first aspect of the present disclosure, there is provided an electronic device for sensing a spectral response from a skin; the device comprising:
- an emitter configured for emitting broadband light towards the skin;
- an array of thin-film microcavity detectors configured for detecting at least two different respective narrowband portions of the broadband light after transmission through or reflection from the skin; wherein each narrowband portion spans a smaller wavelength range than the broadband light.

It will be understood that the emitter is a light emitter since it is configured for emitting light, and that the detectors are light detectors (a.k.a. photodetectors) since they are configured for detecting light.

By comprising an array of thin-film microcavity detectors, the electronic device benefits from multiple on-chip detectors sensitive to different wavelengths, and thus can be integrated, which may help to achieve the above-mentioned advantages of miniaturization, improved performance, and increased efficiency. In other words, the electronic device may have a smaller system size, lower cost, and less power consumption than various known PPG solutions.

Hence, instead of having multiple separate LEDs emitting different wavelengths, as in various known solutions, the electronic device can feature a single chip with an array of PDs sensitive to different wavelengths. The corresponding broadband emitter can be provided on the same chip as the detectors in embodiments using the reflection mode of operation.

Some known devices use interference filters overlaying one or more optical sensors and are used with conventional detectors such as silicon and InGaAs. Differently from such known interference-filter devices, embodiments according to the present invention may achieve filtering with (implicitly resonant) microcavities and detection with thin film, both of which are more advantageous in terms of detector choice. This is because Si has only a limited spectral range (e.g. no SWIR), whereas InGaAs is expensive. With the array of thin-film microcavity detectors, the presently-disclosed electronic device can provide filtering and access to SWIR in a more cost-effective manner.

Furthermore, by comprising an array of thin-film microcavity detectors configured for detecting at least two different respective narrowband portions of the broadband light after transmission through or reflection from the skin, it may be possible to select a high number of separate narrowband portions at the detector side. This may in particular (but not necessarily exclusively) follow from the metasurface-based design of the thin-film microcavity detectors.

For the avoidance of doubt, it is noted that the presently-described thin-film microcavity detectors can be implemented as a Faby-Perot filter, or in other words, as a photosensitive element configured between an upper reflective element and a lower reflective element, as described throughout (and claimed) in EP 4279885 A1.

Additionally, only one or a few LEDs may be needed on the light emitter side, because of its broadband emission spectrum or their broadband emission spectra.

All in all, this allows for wafer-scale integration of multispectral photodetectors directly on readout ICs (integrated circuits), which is beneficial for cost, scaling, and performance.

Lastly, it will be understood that the feature that each narrowband portion spans a smaller wavelength range than the broadband light imposes a relationship between the light emitted by the light emitter (which is broadband, compared to the narrowband) and the light portions detected by the light detectors (which are narrowband, compared to the broadband).

In various embodiments, the array of detectors comprises a plurality of quantum dots arranged in the respective microcavities.

An advantage is that quantum dots provide a cost-effective way of reaching wavelengths of more than 1 um, to improve the applicability of the electronic device for biosensing.

In various embodiments, the plurality of quantum dots (QDs) are of different sizes and have different absorption onsets.

An advantage is that this allows the electronic device to provide a more uniform absorption profile. The biggest QD in the mixture determines the longest wavelength possible to reach, while the other QDs help to smoothen the absorption curve.

In various embodiments, the emitter comprises any of the following: a lamp, preferably a halogen lamp; one or more LEDs, preferably III-V LEDs; a quantum dot thin film; an organic thin film; and a downconverter comprising quantum dots and/or a phosphorus-based material.

An advantage is that this allows for a wide range of possible light emitters.

In various embodiments, at least one of the emitter and the array of detectors is configured to, in operation, contact the skin.

An advantage is that the electronic device can thus be operated non-invasively.

It will be understood that 'contacting' the skin in this context is to be seen as being in physical proximity with, and abutting against, the skin, without piercing the skin.

A further advantage is that the electronic device can thus be safely and reliably operated by an end-consumer, without requiring expert intervention or supervision.

In various embodiments, the emitter is configured for emitting the broadband light at a wavelength full width at half maximum, FWHM, of at least 100 nm, and wherein each detector is configured for detecting the respective narrowband portion at a wavelength FWHM of at most 50 nm.

An advantage is that a higher spectral resolution can be obtained, which in turn enables detection of more biomarkers and/or measurements with higher accuracy.

In various embodiments, the emitter is configured for emitting broadband light spanning the short-wavelength infrared, SWIR, range.

An advantage is that SWIR light is especially useful for biosensing.

In particular, spanning the SWIR range enables monitoring of more parameters, such as - for example - lipids, collagen, water, cholesterol, different proteins, ethanol, glucose, etc. Moreover, spanning the SWIR range is advantageous because in this range there is a relatively low melanin absorption, which helps the light to penetrate deeper into melanin-containing tissue. A further advantage is thus that the electronic device may be made more robust for different skin pigmentations, which may help to broaden applicability and reliability.

In various embodiments, the emitter comprises a plurality of light emitting elements configured to emit light at a respectively corresponding plurality of distinct partially overlapping wavelength ranges simultaneously (i.e. in a time-parallel manner of operation).

An advantage is that the illumination area can be increased, the total power can be increased, and the emission spectrum can be made broader.

Note that known devices use emitters that are operated in a time division multiplexing manner, which fundamentally differs from a simultaneous mode of operation.

In various embodiments, the array of detectors comprises a plurality of detectors arranged to detect a respectively corresponding plurality of distinct narrowband portions of the broadband light.

An advantage is that each respective detector can be tailored to its respective narrowband portion, and thus be made to operate with high efficiency.

In various further-developed embodiments, the plurality of detectors is arranged to detect a respectively corresponding plurality of distinct partially overlapping narrowband portions of the broadband light.

An advantage is that the plurality of detectors can thus cover a continuous wavelength range, to better capture data.

In various embodiments, the array of detectors is configured for detecting the respective narrowband portions of the emitted broadband light simultaneously (i.e. in a time-parallel manner of operation).

An advantage is that the device can therefore be operated in a continuous manner, allowing for a more rapid, more detailed, and more accurate final readout.

In various embodiments, the array of detectors extends in a two-dimensional plane.

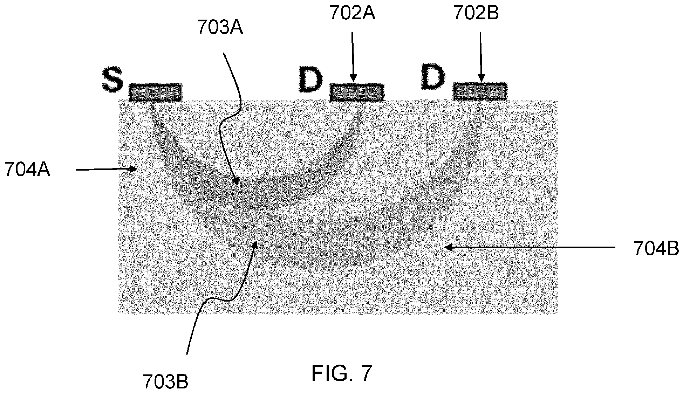

A first advantage is that incident light can be efficiently captured. However, a second advantage is that a two-dimensional (2D) array of detectors allows for multi-channel measurements. Multi-channel measurements allow for probing different tissue regions located at different depths. If a detector is farther away from a light emitter, it can detect light that went deeper into the tissue (as will be explained below with reference to Figure 7). This is relevant because different tissue regions of interest can be located at different positions and/or depths. Hence, the 2D array approach allows for more robust and more accurate measurements. In addition, arrays of detectors allow for reduction of motion artefacts that corrupt the measurements.

It is thus noted that an array of detectors facilitates multi-wavelength measurements, already when the array of detectors is one-dimensional (1D), e.g. a row of detectors. Expanding the array of detectors to two dimensions (2D) facilitates simultaneous multi-wavelength and multi-channel measurements. In an exemplary embodiment, one dimension may be used for detecting different wavelengths, and the other may be used to detect the same wavelength for different positions.

In various embodiments, the array of detectors is monolithically integrated on a chip for readout of signals from the array of detectors.

An advantage is that the electronic device can be small and cost-efficient.

In various embodiments, the array of detectors is cointegrated with meta-surfaces in the microcavities, in order to control their spectral response.

An advantage is that this allows for an efficient tunning of microcavities: one meta-surface layer may in principle enable tunning of the microcavities to an infinite number of different wavelengths.

The metasurface-based design means that there is a metasurface layer inside the microcavity, together with the thin-film photodetector, and the metasurface tunes the detection wavelength, i.e. the resonant condition of the cavity. Hence, with a single flat metasurface layer it is possible to tune the resonant cavity (more precisely: its optical thickness) to a high number (in theory, infinite) of resonant wavelengths.

In various embodiments, the emitter and the array of detectors are embedded in a surface of a visual display of the electronic device.

An advantage is that the electronic device can be used to both display a visual interface configured to receive a user's skin portion and sense the skin portion's spectral response. A particular example of this may include a skin sensor incorporated into a display of a smart device, such as a smartphone or a smartwatch, or incorporated into a display of an access device, such as a turnstile access screen.

Further developed embodiments may for example be configured to detect and recognize vein patterns in the skin, to provide authentication.

In various embodiments, the emitter is configured for emitting broadband light covering a wavelength range from 900 nm to 2500 nm.

An advantage is that this allows for detection of more biomarkers which have pronounced absorption features at longer wavelengths.

It is noted that for Si the lower limit is around 1100 nm, but embodiments according to the present disclosure are not necessarily limited to Si material.

In various embodiments, the emitter is arranged on an at least SWIR-transparent substrate, and said substrate is arranged on the array of detectors, and said substrate comprises a plurality of collimators configured to guide and restrict the respective narrowband portions of the emitted broadband light, after said light has been transmitted through or reflected from the skin, towards the array of detectors.

An advantage is that this obviates the need for a separate lens, further improving miniaturization of the electronic device.

In various embodiments, the electronic device may comprise at least one reflector arranged to be, in operation of the electronic device, between the emitter and the skin; wherein the at least one reflector is receptive to the emitter and is arranged to reflect incoming light towards the array of detectors, such that at least a part of the broadband light is reflected towards the array of detectors without reaching the skin.

An advantage is that the one or more reflectors may provide a reflected signal that has not interacted with the skin, thus offering a baseline signal for calibration. This in turn may help to make the electronic device more resilient against various changes in operating conditions, e.g. environmental humidity, skin humidity, skin oiliness, skin cracking, environmental temperature, etc.

In various embodiments, the skin is human skin.

In various embodiments, the device is wearable.

An advantage is that this allows the electronic to be used "in the field" by the end-consumer, which increases the overall applicability of the device.

According to a second aspect of the present disclosure, there is provided a method of sensing a spectral response from a skin; the method comprising:
- emitting broadband light towards the skin;
- detecting at least two different respective narrowband portions of the broadband light, after transmission through or reflection from the skin; wherein each narrowband portion spans a smaller wavelength range than the emitted broadband light;
wherein the step of detecting is performed using an array of thin-film microcavity detectors.

It will be understood by the skilled person that various specific features, advantages, and technical considerations applying to the above-described embodiments of the electronic device according to the present disclosure may also apply to embodiments of the method, *mutatis mutandis.* In particular, any device embodiment described above or below in the present disclosure will be understood by the skilled person to have or potentially have a corresponding method embodiment, and *vice versa.*

### Brief description of the drawings

For a better understanding of the present disclosure, some exemplary embodiments are described below in conjunction with the appended figures and figures description, wherein:
Figure 1 schematically illustrates a reflection mode and a transmission mode of operation.
Figure 2A schematically illustrates different components of collected optical signal.
Figure 2B schematically illustrates absorption spectra of oxygenated and deoxygenated haemoglobin.
Figure 3A schematically illustrates experimental results of blood vessel imaging with IR image sensors experimental results.
Figure 3B schematically illustrates an optical imaging system relating to the present disclosure, and light propagation through skin tissue.
Figure 4 schematically illustrates a system diagram of an exemplary spectrometer.
Figure 5A schematically illustrates an example of a known time-multiplexing approach for non-invasive optical biosensing.
Figure 5B schematically illustrates an example application of an electronic device according to the present disclosure.
Figure 6A schematically illustrates a plot graphing normalized emission spectra of SWIR LEDs available commercially.
Figure 6B schematically illustrates a plot graphing a sequence of narrowband portions detectable by the array of photodetectors of an electronic device according to the present disclosure.
Figure 7 schematically illustrates an example of multi-channel measurement involving probing different tissue regions located at different depths by means of detectors at different locations in the array of a device embodiment according to the present disclosure.
Figure 8 schematically illustrates an embodiment of the electronic device according to the present disclosure, which can be used for under-skin imaging and spectral measurements.
Figure 9 schematically illustrates various embodiments of the electronic device according to the present disclosure, showing how the emitter and (not shown) the array of detectors can be embedded in a surface of a visual display of the electronic device.

### Detailed description of certain illustrative embodiments

The present disclosure will be described with respect to particular embodiments and with reference to certain drawings but the disclosure is not limited thereto. The drawings described are only schematic and are non-limiting. In the drawings, the size of some of the elements may be exaggerated and not drawn on scale for illustrative purposes. The dimensions and the relative dimensions do not necessarily correspond to actual reductions to practice of the disclosure.

Furthermore, the terms first, second, third and the like in the description and in the claims, are used for distinguishing between similar elements and not necessarily for describing a sequential or chronological order. The terms are interchangeable under appropriate circumstances and the embodiments of the disclosure can operate in other sequences than described or illustrated herein.

Moreover, the terms top, bottom, over, under and the like in the description and the claims are used for descriptive purposes and not necessarily for describing relative positions. The terms so used are interchangeable under appropriate circumstances and the embodiments of the disclosure described herein can operate in other orientations than described or illustrated herein.

The term "comprising", used in the claims, should not be interpreted as being restricted to the means listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting of only components A and B. It means that with respect to the present disclosure, the only relevant components of the device are A and B.

An example of an array of thin-film microcavity detectors that would be suitable for embodiments of the electronic device according to the present disclosure can be found in published patent application EP 4279885 A1, titled "A light sensor and a method for manufacturing a light sensor". Said published application presents various suitable embodiments of such an array over its entire contents, and in particular from paragraph 112 onwards until paragraph 238, with reference also to its drawings.

The claimed light sensor of EP 4279885 A1 comprises an upper reflective element and a lower reflective element, and a photo-sensitive element arranged between the upper and lower reflective elements. As is explained in EP 4279885 A1, the photo-sensitive element may be formed using thin-film technology.

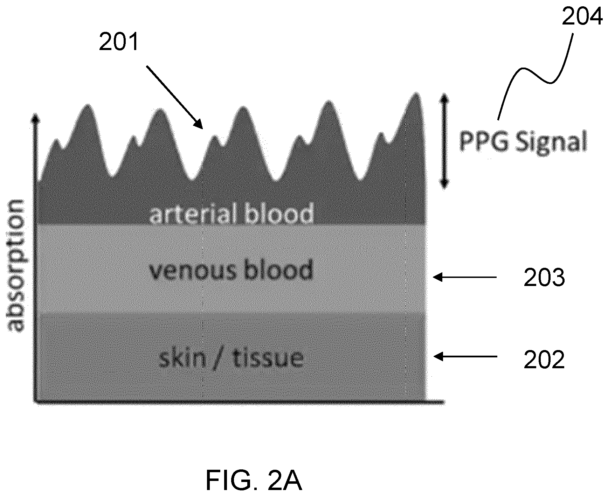

Photodetectors collect signals that consist of a so-called DC (because it is relatively stable, like direct current, DC) component that originates from venous blood or tissue and a weaker so-called AC (because it fluctuates, like alternating current, AC) PPG signal that originates from pulsating arterial blood, as is illustrated in Figure 2A. By analysing the transient signal different parameters relevant to cardiological health can be extracted, including heart rate.

Figure 2A schematically illustrates different components of collected optical signal.

The figure shows an absorption spectrum 201 defined by the superposition of a skin or tissue component 202, a venous blood component 203, and a PPG signal 204, mostly due to arterial blood (which is of course subject to heart-driven circulation and variations).

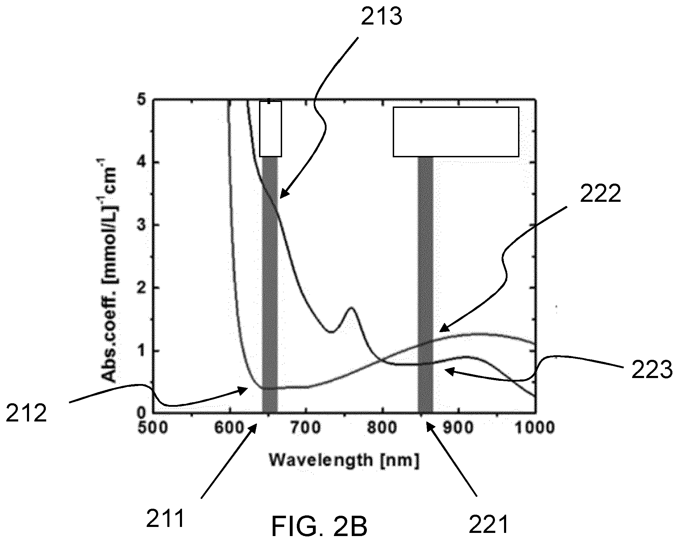

Figure 2B schematically illustrates absorption spectra of oxygenated and deoxygenated haemoglobin.

The figure shows how a first wavelength 211 around 650 nm can be used to detect a higher absorption coefficient for oxygenated blood 212 than for deoxygenated blood 213, whereas a second wavelength 221 around 850 nm can be used to detect a higher absorption coefficient for deoxygenated blood 223 than for oxygenated blood 222. In this manner, blood oxygenation can be more accurately detected using multiple different wavelengths.

In addition, PPG sensors operate as spectroscopic devices that utilize LEDs that emit at different wavelengths and rely on certain unique absorption features of molecules of interest for their detection. LEDs used in PPG sensors commonly emit red (usually 660 nm) and near-infrared (NIR, usually 940 nm, but around 850 nm would also work well) light because deoxygenated haemoglobin in blood exhibits higher light absorption in red, whereas oxygenated haemoglobin is more absorptive in NIR, as is illustrated in Figure 2B. By comparing light attenuation in these two spectral ranges oxygenation levels can be obtained.

In general, there is a trend and motivation to extract more information and quantify different parameters in a continuous and non-invasive way. Some of the parameters of interest include hydration, cholesterol, triglycerides, lactate, different proteins, glucose, ethanol, etc. Optical continuous and non-invasive sensors that can detect multiple parameters beyond blood oxygenation are already available on the market and they are typically used in hospitals. For example, continuous non-invasive measurements of total haemoglobin are done for an early detection of occult bleeding or for an early warning in anaemic patients. Detection of various parameters such as lipids (e.g. cholesterol) and lactate has been demonstrated in the literature but has not reached the market yet. Many attempts have been made to measure blood glucose in a similar way, but with limited success so far.

In order to expand the number of detectable and quantifiable physiological parameters, optical sensors need to have more LEDs operating at different wavelengths, which increases the amount of information that can be collected. Depending on the parameters of interest, different wavelengths are selected to target specific absorption features. For example, different types of PPG sensors are produced that employ different numbers of LEDs: two LEDs enable standard measurement of oxygen saturation, four LEDs additionally enable measurements of various (proprietary) indices, and eight LEDs further add a possibility to measure Total Hemoglobin (SpHb), Carboxyhemoglobin (SpCO), Methemoglobin (SpMet) and Oxygen Content (SpOC). The LEDs typically employed emit wavelengths ranging from visible to near-infrared (NIR). The longer wavelengths are beneficial for biomarker sensing because many biological molecules found in human tissue and blood exhibit specific absorption in NIR/SWIR, and light at longer wavelengths penetrates deeper into tissue due to reduced scattering. One prominent absorbent in SWIR is water. Some devices feature skin detectors which use 1050 nm and 1450 nm LEDs to detect on-skin contact. Moreover, a watch is known with an optical sensor for measuring a hydration index.

In addition to increasing the number of detectable parameters, adding more LEDs with different emission wavelengths also increases the accuracy of measurements. For example, it has been reported that the accuracy of non-invasive glucose measurements with optical SWIR sensors increases from 25-30 mg/ml to 20 mg/ml by using three SWIR LEDs (1600-1700 nm range) instead of two. A multiwavelength approach has also been applied to eliminate motion artefacts that corrupt collected signals.

As explained above, by increasing the number of LEDs it is possible to increase the number of detectable physiological parameters as well as to improve the measurement accuracy. However, standard LEDs are available as discrete components and with each additional LED the cost and the size of the system increases. A known sensor (mentioned above) which features 8 LEDs is almost three times more expensive than their sensor featuring 4 LEDs. At a certain point, it becomes unpractical to add more LEDs due to cost and size constraints.

The variety of LEDs in the SWIR range is limited. For example, a known vendor offers 9 different LEDs in the 1000-1700 nm range, of which only 3 cover the entire 1400-1700 nm range. Other vendors offer similar products in terms of spectral coverage. Clearly, conventional SWIR LEDs available on the market cannot provide enough spectral resolution. Custom LEDs would be needed to target specific wavelengths of interest for detection of various molecules, which would further drive the system cost up.

The emission spectrum of conventional LEDs is relatively broad, especially in SWIR, which further limits the resolution of spectroscopic measurements. For example, SWIR LEDs from some vendors have FWHM in the range of 50-150 nm within the 1300-1700 nm spectral region. As discussed already, many molecules exhibit unique features in SWIR (e.g. water, lipids and glucose) but since tissues and bodily fluids contain a wide variety of different molecules, their absorption spectra overlap. Hence, a higher spectral resolution with narrower emission bandwidth and a higher number of separate bands are needed for detection and quantification of a higher number of physiological parameters.

Finally, a challenge associated with the photodetection side can be observed as well. For a collection of a robust signal and implementation of various noise cancellation methods, multiple photodetectors need to be used, often arrays of photodetectors. Arrays of photodetectors can be used for signal averaging. In addition, multiple photodetectors enable detection of light that travels via different absorption paths through a tissue which is important for user-to-user data normalization and for elimination of contribution of unknown absorbents on skin (e.g. lotions or sweat). Like LEDs, each additional photodetector increases the price of the system, especially if SWIR photodetectors need to be used.

### Problems of Si photonics for spectral biomarker sensing

The problems of the conventional optical biomarker sensing based on LEDs are recognized in the industry and academia and several companies are trying to overcome them by using SWIR lasers as emitters instead of LEDs. Some of these companies reported accurate measurements of glucose using bulky benchtop spectrometers. The technology of choice in those cases seems to be silicon photonics. However, this technology brings a completely new set of problems, namely that it is challenging to integrate many lasers on 200 mm or 300 mm Si wafers with high yield and at a low cost.

Guiding horizontal light flow in integrated photonics may involve a complicated set of optical components such as multiplexers, gratings and waveguides for out-coupling and in-coupling of light for interaction with a measured medium. Each of these components reduces the SNR (signal-to-noise ratio) due to unavoidable optical losses. Furthermore, a spectrometer photonic integrated circuit (PIC) typically needs to be bonded to a CMOS ASIC (complementary metal-oxide semiconductor application-specific integrated circuit), which may complicate the integration procedure and may thus increase the overall system cost. Additionally, many photodetectors based on Si photonics tend to suffer from high noise levels leading to a (further) degradation of the system SNR.

### Complex light interaction with skin tissue

In general, a major challenge with on-skin spectroscopic measurements is a complex tissue composition which affects light in various different and hard to predict ways. Light is scattered, reflected and absorbed by a complex mixture of molecules and cells. For accurate measurements it is important to know the path that light travels, which is not trivial. Many attempts have been done to model the optical properties of skin tissue, but most of the time the approach is empirical in nature and algorithms are developed to corelate the received light to a concentration of a specific molecule found in blood or interstitial fluid.

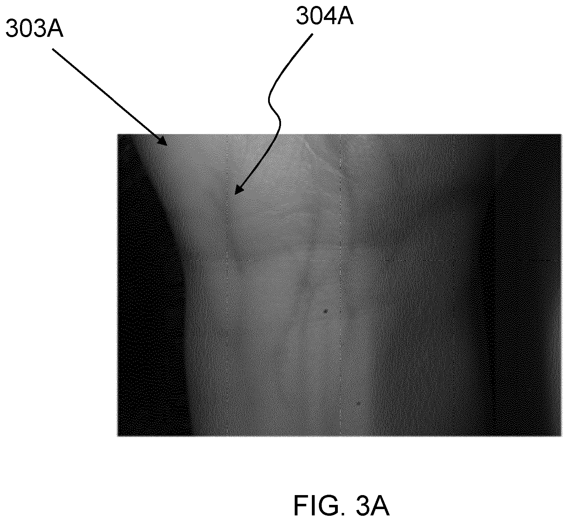

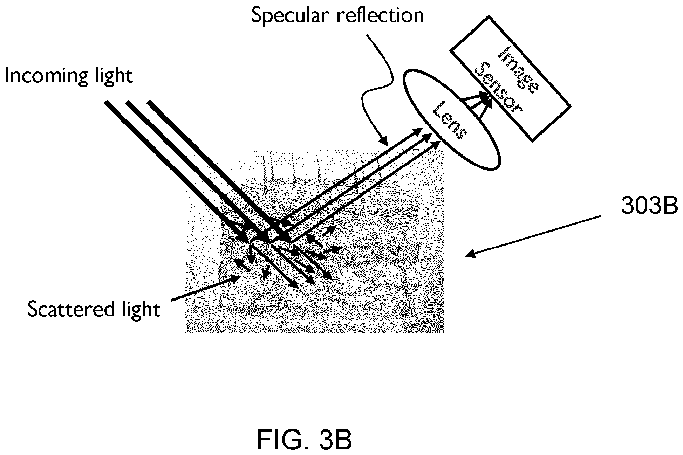

On the other hand, it is believed that IR imaging can reveal structures below the skin surface (e.g. veins and capillary, as illustrated in Figure 3A). Light that enters the tissue is not only scattered and absorbed but also reflected in a specular manner which can be collected (for example via a lens) and used for imaging the inner composition of the tissue (as illustrated in Figure 3B).

Light reflected in a specular manner has a more predictable path through the tissue and hence, it is more suitable for spectroscopic measurements and quantification of different parameters. However, a certain distance is usually necessary between the object and the lens for an imaging system, as well as between the lens and the image sensor. For applications described here, compactness is important. Therefore, an imaging system with a conventional lens is less preferred.

Some embodiments according to the present disclosure may therefore be based *inter alia* on collimators that allow for imaging in a small compact form, in conjunction with broadband emission and spectrally resolved arrays of photodetectors.

Figure 3A schematically illustrates experimental results of blood vessel imaging with IR image sensors experimental results. In the figure, a part of a human hand 303A is shown, indicating the visibility of veins 304A below the skin surface, detected under NIR light.

Figure 3B schematically illustrates an optical imaging system 303B relating to the present disclosure, and light propagation through skin tissue.

The figure shows incoming light being scattered as scattered light by the skin tissue, and then propagating via specular reflection towards a lens, which is arranged to bundle and focus said reflected light towards an image sensor.

### Reference problem

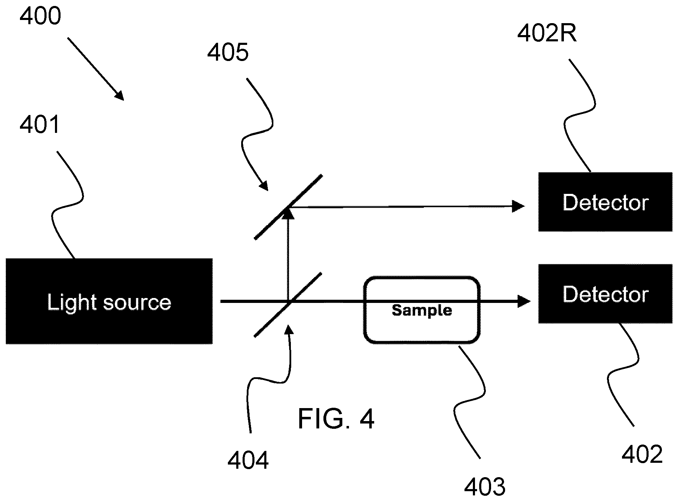

Another problem regarding miniaturized spectroscopic sensors is that a reference light source needs to be provided in order to perform quantifications of the absorbent of interest, which is challenging to achieve in a compact form. In conventional spectroscopic measurements, this is done for example with beam splitters and mirrors which pass one portion of the light source directly to the detector, whereas the other beam passes through the sample, as is illustrated in Figure 4. Integrated photonic platforms (e.g. Si photonics) provide a mean to achieve this by using integrated beam splitters and waveguides.

Figure 4 schematically illustrates a system diagram of an exemplary spectrometer 400 intended to alleviate this reference problem. Such a spectrometer is an example of an electronic device according to the present disclosure.

The figure shows a light source 401 configured for emitting light towards a sample 403. The emitted light may be split 404 between the light source 401 and the sample 403, for example using an optical splitter or simply using a spatially separated reflector 405. The light that has interacted with sample 403 may be detected by detector 402, whereas the light that has not interacted with the sample may be detected by reference detector 402R. In this manner, the device provides access to a reference signal, which can be used in various manners.

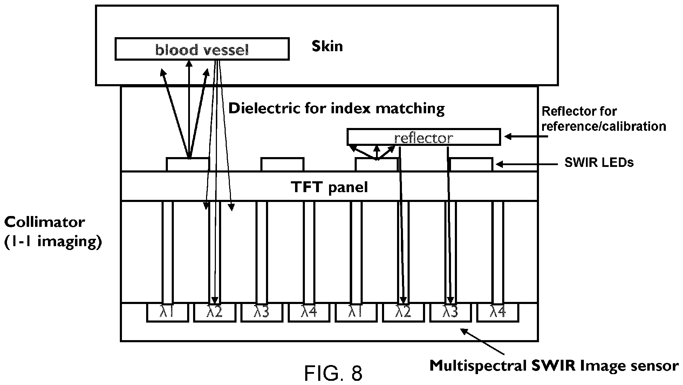

Reference is further made to Figure 8 below, which also features a reference reflector.

Embodiments according to the present disclosure may find application for spectroscopic measurements, particularly for measuring biomarkers in skin tissue and blood. Particular embodiments according to the present disclosure relate to an electronic device for PPG. The device may be useful for optical sensing and imaging, and may enable higher spectral resolution compared to known systems based on time-multiplexing of LEDs (according to the reflection mode 100A or transmission mode 100B as illustrated in Figure 1).

A specific exemplary use case may involve checking for water in the skin tissue of the ear to detect inflammation.

### Non-Invasive Sensing with Arrays of Photodetectors

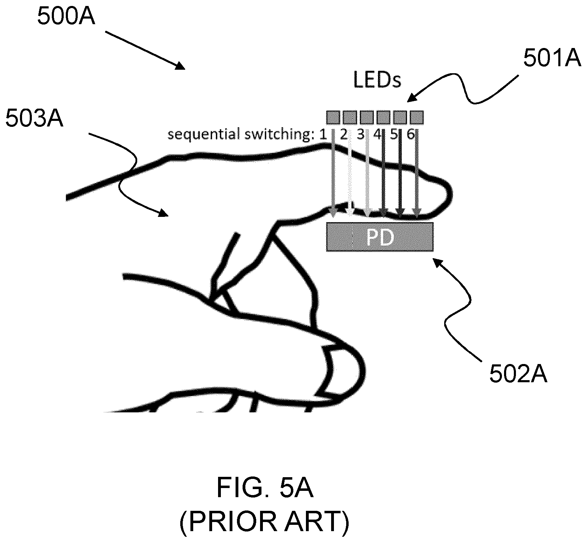

Figure 5A schematically illustrates an example of a known time-multiplexing approach for non-invasive optical biosensing.

In known optical sensors, as described in the background section hereinabove, spectral information may be encoded on the light emitter side. This means that each light emitter may emit light within a relatively narrow band of wavelengths (e.g. with a full width at half maximum, FWHM, in the range of 50-150 nm for SWIR LEDs). In this setup, a single LED from a group of LEDs may be active at any one time, and distinct LEDs from said group may be individually switched on in a sequential order 1-2-3-4-5-6, as is illustrated in Figure 5A.

In other words, the operation of such known optical sensors may be as follows:
a. first LED1 may be activated;
b. subsequently LED1 may be deactivated and LED2 may be activated;
c. subsequently LED2 may be deactivated and LED3 may be activated;
d. subsequently LED3 may be deactivated and LED4 may be activated;
e. subsequently LED4 may be deactivated and LED5 may be activated;
f. subsequently LED5 may be deactivated and LED6 may be activated;
g. subsequently LED6 may be deactivated;
h. after the deactivation of LED6, the cycle may be repeated from step 1 or this operation may be paused or halted.

The light attenuated by (a portion of) body tissue may be detected by a broadband photodetector (for reasons of simplicity and cost), and signals originating from different LEDs may be separated by their wavelength, due to the time-multiplexing mode of operation.

In addition to the above-described drawbacks, such an approach proves cumbersome once the number of subsequent LEDs rises. Moreover, such an approach poses high requirements of alignment, to increase robustness against various environmental factors (such as sweat, lotions, etc.), but also against various other noise sources.

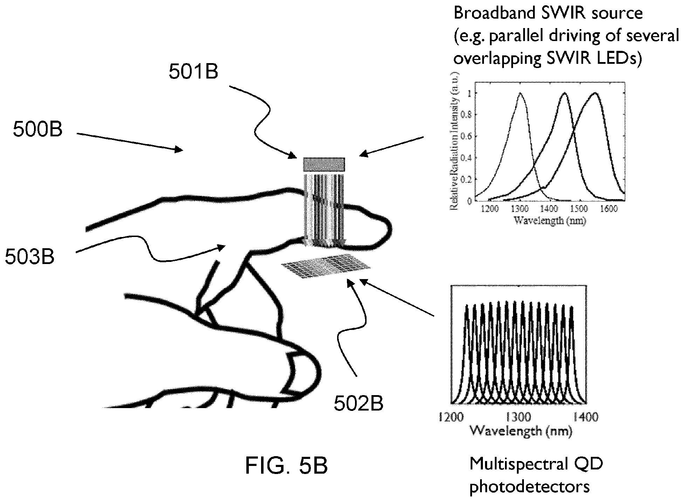

Figure 5B schematically illustrates an example application of an electronic device according to the present disclosure.

In contrast to the known optical sensors described above, various embodiments of the electronic device according to the present disclosure may find application (for example as illustrated in Figure 5B) in a different mode of operation based on a broadband emitter that simultaneously emits light within a broader range of wavelengths (e.g. FWHM >150 nm or higher) and spectral information is obtained on the detection side, as is illustrated in Figure 5B. Each photodetector within an array of photodetectors is responsive to a narrow spectral range (e.g. FWHM < 50 nm, FWHM < 30 nm, or FWHM < 20 nm). Instead of doing conventional time-multiplexing of light emitters, the photodetectors filter the incoming broadband light, operating in parallel. In other words, each individual photodetector of the plurality of photodetectors may be configured to be responsive to a specific narrow spectral range and to reject (i.e. block or attenuate) other wavelengths outside of that specific narrow spectral range.

Exemplary advantages of various embodiments according to the present disclosure may include the following:
By employing microcavity photodetectors with a narrowband response, the spectral resolution can be significantly improved. the optical response of the detectors can be designed to select a narrower range of wavelengths compared to the emission spectra of commercial LEDs.

An array of spectrally sensitive photodetectors can be made monolithically in a CMOS foundry on a readout ASIC. As discussed hereinabove, having multiple photodetectors may facilitate extraction of a more robust signal and implementation of different noise canceling methods. Monolithic integration also enables compact form factor and low cost.

Monolithic integration may also allow that the number of collected bands can be increased more easily.

Since the signals for all the bands can be collected simultaneously without time-multiplexing, the measurements can be performed faster and continuously.

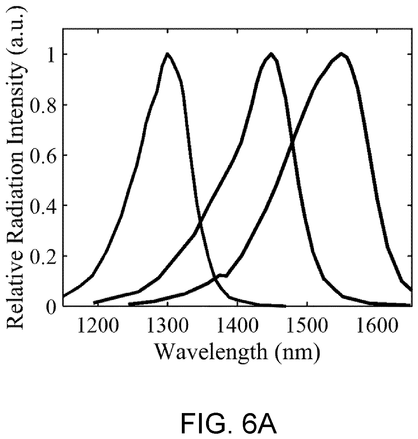

By simultaneously driving several LEDs it is possible to achieve broadband emission with high power. Emission spectra of commercially available SWIR LEDs (as is illustrated in Figure 6A) may for example overlap to a large degree and together cover a broad range from 1150 nm to 1650 nm. Even a single SWIR LED might be sufficiently broadband for a particular application. The broadband emission spectra of SWIR LEDs that was earlier stressed as a drawback for the time-multiplexing approach is in fact an advantage in this mode of operation since a small number of different LEDs is sufficient to cover a broad spectrum. Optionally, an array of LEDs can be simultaneously used to increase the illuminated area and total power. Instead of conventional LEDs, emissive CQDs (Colloidal Quantum Dots, e.g. PbS, PbSe, InAs, InAsP, InSB) can be employed, either in electroluminescent mode or in photoluminescent mode as optical down-converters. Using CQDs of different sizes can enable broadband emission and may even facilitate optional calibration (because then the sampling signal and the reference signal can originate from close to each other).

Figure 6A schematically illustrates a plot graphing normalized emission spectra of SWIR LEDs available commercially.

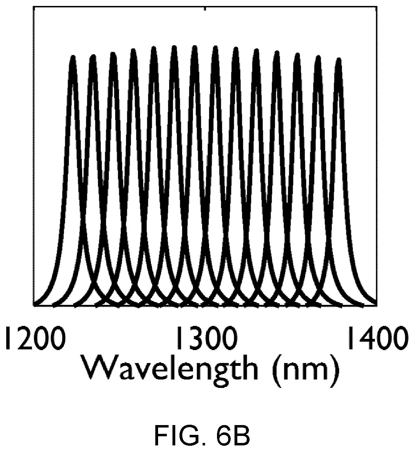

Figure 6B schematically illustrates a plot graphing a sequence of narrowband portions detectable by the array of photodetectors of an electronic device according to the present disclosure.

Figure 7 schematically illustrates an example of multi-channel measurement involving probing different tissue regions located at different depths by means of detectors at different locations in the array of a device embodiment according to the present disclosure. If a detector in the array is farther away from an emitting light source (in this example, detector 702B is located farther away from the light source S than is detector 702A), it can detect light 703B that went deeper 704B into the tissue than the light 703A that only went less deep 704A into the tissue. As explained hereinabove, this is relevant because different tissue regions of interest can be located at different positions and/or depths.

Two other problems described herein, namely complex light interaction with skin tissue and the reference problem, may be solved by enabling imaging functionality in close contact. An image sensor is placed below a transparent panel with SWIR emitters (e.g. LEDs on a **TFT** (thin-film transistor) readout panel) with a collimator in-between, as is illustrated in Figure 8. The collimator can be micro-fiber plate or micro-aperture arrays, commonly used in fingerprint sensors. This configuration essentially allows only specular light reflected at a close to normal incidence to be passed to the photodetector array. Such an arrangement is known as 1-1 imaging and it allows imaging without a lens, and in this context can enable imaging of blood vessels below the skin surface at the right wavelengths. As already discussed, specular reflection is desirable for spectroscopic measurements due to its more predictable light path. The reference problem discussed in the previous section is solved by adding a reflective plate which reflects back a portion of the emitted light, to prevent (or reduce) interaction of that portion with the tissue, such that the reflected portion of light can serve as a reference signal. The configuration with the reference reflector described here requires reflection mode of operation of the sensor, i.e. the emitter and the detector need to be on the same side of the tissue.

Additionally, a single-sided device can be used with more diverse skin surfaces (fingers, head, belly, ...) and is more forgiving in the sense that there is less or even no need to align receiver with transmitter.

Figure 8 schematically illustrates an embodiment of the electronic device according to the present disclosure, which can be used for under-skin imaging and spectral measurements.

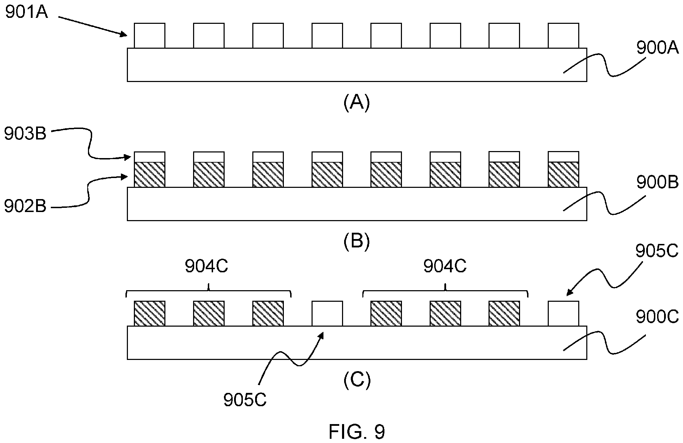

Figure 9 schematically illustrates various embodiments (A, B, and C) of the electronic device according to the present disclosure, showing how the emitter can be embedded in a surface of a visual display of the electronic device.

Figure 9A schematically illustrates that the electronic device may comprise a **TFT** panel 900A on which is disposed a plurality of neighbouring SWIR LEDs 901A (embodiment A). In a first exemplary embodiment, each SWIR LED may comprise a III-V semiconductor device. In a second exemplary embodiment, each SWIR LED may comprise a CQD (Colloidal Quantum Dots) device as explained above. In embodiments like these first and second exemplary embodiments, each LED on the **TFT** panel may be identical.

Alternatively, in a third exemplary embodiment (embodiment B), each LED on the **TFT** panel 900B may comprise a LED 902B operable in the visible light spectrum (e.g. an OLED or a uLED) capped with a SWIR QD (Quantum Dot) converter 903B, as is schematically illustrated in Figure 9B. In embodiments like this third exemplary embodiment, each LED on the **TFT** panel may be identical.

In yet another alternative, in a fourth exemplary embodiment (embodiment C), the electronic device may comprise a TFT panel 900C on which is disposed a plurality of neighbouring LEDs 904C, 905C, comprising sets of LEDs 904C operable in the visible light spectrum (e.g. an OLED or a uLED), separated by SWIR LEDs 905C. In a specific example, each SWIR LED 905C may comprise a III-V semiconductor device. In another example, each SWIR LED 905C may comprise a CQD (Colloidal Quantum Dots) device as explained above. It may even be contemplated technically to use some III-V SWIR LEDs combined with some CQD-based SWIR LEDs, although this would of course increase production complexity and costs. It will of course be understood that in embodiments like this fourth exemplary embodiment, not every LED 904C, 905C on the TFT panel is identical.

### Different embodiments - variations of the light source

SWIR emitters can be realized in different ways. For example, the emitters can be disposed within a display layer, or the emitters can be disposed between a display layer and the array of detectors, or the emitters can be disposed next to the array of detectors on the same plane.

Finally, the skilled person will understand that embodiments according to the present disclosure may find application in PPG, non-invasive sensing, continuous sensing, and biomarker sensing, non-invasive and/or continuous monitoring of biomarkers, and in biometric identification with optional vein pattern recognition.

## Claims

1. An electronic device (500B) for sensing a spectral response from a skin; the device comprising:
- an emitter (501B) configured for emitting broadband light towards the skin;
- an array of thin-film microcavity detectors (502B) configured for detecting at least two different respective narrowband portions of the broadband light after transmission through or reflection from the skin; wherein each narrowband portion spans a smaller wavelength range than the broadband light.

2. The electronic device of claim 1, wherein the array of detectors comprises a plurality of quantum dots arranged in the respective microcavities, and wherein the plurality of quantum dots preferably are of different sizes and preferably have different absorption onsets.

3. The electronic device of claim 1, wherein the emitter comprises any of the following: a lamp, preferably a halogen lamp; one or more LEDs, preferably III-V LEDs; a quantum dot thin film; an organic thin film; and a downconverter comprising quantum dots and/or a phosphorus-based material.

4. The electronic device of any one preceding claim, wherein the emitter is configured for emitting the broadband light at a wavelength full width at half maximum, FWHM, of at least 100 nm, and wherein each detector is configured for detecting the respective narrowband portion at a wavelength FWHM of at most 50 nm.

5. The electronic device of any one preceding claim, wherein the emitter is configured for emitting broadband light spanning the short-wavelength infrared, SWIR, range, preferably covering a wavelength range from 900 nm to 2500 nm.

6. The electronic device of any one preceding claim, wherein the emitter comprises a plurality of light emitting elements configured to emit light at a respectively corresponding plurality of distinct partially overlapping wavelength ranges simultaneously.

7. The electronic device of any one preceding claim, wherein the array of detectors comprises a plurality of detectors arranged to detect a respectively corresponding plurality of distinct narrowband portions of the broadband light, wherein the narrowband portions preferably overlap partially.

8. The electronic device of any one preceding claim, wherein the array of detectors is configured for detecting the respective narrowband portions of the emitted broadband light simultaneously.

9. The electronic device of any one preceding claim, wherein the array of detectors extends in a two-dimensional plane.

10. The electronic device of any one preceding claim, wherein the array of detectors is monolithically integrated on a chip for readout of signals from the array of detectors.

11. The electronic device of any one preceding claim, wherein the array of detectors is cointegrated with metasurfaces in the microcavities, in order to control their spectral response.

12. The electronic device of any one preceding claim, wherein the emitter and the array of detectors are embedded in a surface of a visual display of the electronic device.

13. The electronic device of any one preceding claim, wherein the emitter is arranged on an at least SWIR-transparent substrate, and wherein said substrate is arranged on the array of detectors, and wherein said substrate comprises a plurality of collimators configured to guide and restrict the respective narrowband portions of the emitted broadband light, after said light has been transmitted through or reflected from the skin, towards the array of detectors.

14. The electronic device of any one preceding claim, comprising at least one reflector arranged to be, in operation of the electronic device, between the emitter and the skin; wherein the at least one reflector is receptive to the emitter and is arranged to reflect incoming light towards the array of detectors, such that at least a part of the broadband light is reflected towards the array of detectors without reaching the skin.

15. A method of sensing a spectral response from a skin; the method comprising:
- emitting broadband light towards the skin;
- detecting at least two different respective narrowband portions of the broadband light, after transmission through or reflection from the skin; wherein each narrowband portion spans a smaller wavelength range than the emitted broadband light;
wherein the step of detecting is performed using an array of thin-film microcavity detectors.
